# EUROPÄISCHE PATENTSCHRIFT

(11) **EP 1 181 089 B1**
(45) Veröffentlichungstag und Bekanntmachung des Hinweises auf die Patenterteilung: **10.11.2004**
(21) Anmeldenummer: 00925238.8
(22) Anmeldetag: 26.04.2000
(51) Int. Cl.: B01D 61/24, B01D 11/04

(54) **VERFAHREN ZUR ABTRENNUNG ORGANISCHER SUBSTANZEN AUS EINEM WÄSSRIGEN GEMISCH**
METHOD FOR SEPARATING ORGANIC SUBSTANCES FROM AN AQUEOUS MIXTURE
PROCEDE DE SEPARATION DE SUBSTANCES ORGANIQUES CONTENUES DANS UN MELANGE AQUEUX

(30) Priorität: 29.04.1999 DE 19919490
(43) Veröffentlichungstag der Anmeldung: 27.02.2002
(73) Patentinhaber: DSM Biotech GmbH, 52428 Jülich (DE); DSM IP Assets B.V., 6411 TE Heerlen (NL); Forschungszentrum Jülich GmbH, 52425 Jülich (DE)
(72) Erfinder: MAASS, Dietrich, D-48341 Altenberge (DE); WEUSTER-BOTZ, Dirk, D-85221 Dachau (DE); TAKORS, Ralf, D-52399 Merzenich (DE); WANDREY, Christian, D-52428 Jülich (DE); PASCHOLD, Holger, D-52441 Linnich (DE)
(74) Vertreter: Jacobs, Monique S.N.
(86) Internationale Anmeldenummer: PCT/EP2000/003705
(87) Internationale Veröffentlichungsnummer: WO 2000/066253

(56) Entgegenhaltungen:
- WO-A-93/23150
- J. OF MEMBRANE SCIENCE, Bd. 58, 1991, Seiten 11-32, XP000248958 in der Anmeldung erwähnt

## Beschreibung

Die vorliegende Erfindung betrifft ein Verfahren zur Abtrennung von einer oder mehreren in einem wäßrigen Gemisch vorhandenen organischen Substanzen enthaltend zumindest eine positiv geladene und/oder aufladbare stickstoffhaltige Gruppe mittels Extraktion über wenigstens eine poröse Membran.

Techniken zur Separation organischer Verbindungen aus wässrigen Lösungen sind zahlreich bekannt. Hierzu zählen beispielsweise eine Auftrennung über Ionenaustauscherharze, Chromatographieverfahren, Adsorption, Filtration, Evaporation, reverse Osmose, Elektrodialyse usw.

Insbesondere die Gewinnung von Aminosäuren hat dabei in den letzten Jahren vor allem mit Blick auf die Lebensmittel- und Getränkeindustrie wirtschaftlich an Interesse zugenommen. Die Separation der gewünschten Aminosäuren aus Gemischen erfolgt z. B. vor allem über Ionenaustauscher oder beispielsweise mittels reaktiver Extraktion. Diese Verfahren stoßen jedoch bei der Aufreinigung insbesondere von Kulturbrühen an ihre Grenzen. Nachteilig bei einer Behandlung von Kulturflüssigkeiten über Ionenaustauscher ist das Erfordernis einer extensiven Vorbehandlung des zu reinigenden Gemisches.

Insbesondere für die Aufreinigung von L-Phenylalanin sind Extraktionen über verschiedene flüssige Membranen beschrieben (Thien, M.P., et al. Biotechnol Bioeng,1998, 32: 604-615). Hierbei handelt es sich im weitesten Sinne um Öl-in-Wasser-Emulsionen unter Ausbildung von organischen, beispielsweise Lipidhaltigen Membranvesikeln, über die dann die eigentliche Extraktion erfolgt. Solche flüssig-flüssig Extraktionen weisen allerdings bei hohen Produktkonzentrationen Limitationen durch ein erhebliches Anschwellen der Membranvesikel auf. Ferner ist die Stabilität der Membranvesikel gegenüber Drücken kritisch.

Dispersionsfreie Extraktionsverfahren über Hohlfasern zur Separation von L-Phenylalanin sind bereits bei Escalante H. et al. (1998, Separation Science and Technology, 33(1): 119-139) beschrieben. Als Extraktionsmittel wird hier ein Gemisch aus quarternären Ammoniumsalzen, Isodecanol und Kerosin verwendet, das sich allerdings durch eine Selektivität gegenüber Anionen auszeichnet. Der Nachteil dieses Verfahrens besteht darin, daß für die Extraktion von L-Phenylalanin eine Titration des Mediums auf einem pH-Wert von 10,5 erforderlich ist. Für die Rückextraktion aus der organischen Phase ist eine weitere Titration auf einen pH-Wert von 0,5 notwendig. Zusätzlich verhindert die Toxizität des Extraktionsmittels, insbesondere der quarternären Ammoniumsalze, die auch als Desinfektionsmittel eingesetzt werden, eine direkte Rückführung des Mediums in den Produktionsprozeß. Ein weiterer Nachteil dieses Verfahrens besteht darin, daß die Abtrennung des L-Phenylalanins nicht in den Fermentationsprozeß integriert werden kann und somit nicht die Möglichkeit besteht, eine potentielle Produktinhibierung zu verhindern.

Die Extraktion von Aminosäuren über ein Kationen-selektives Extraktionsmittel, bestehend aus Di-2-Ethyl-Hexyl-Phosphorsäure gelöst in n-Decanol, ist bei Teramoto M. et al. (1991, J Membr Sci, 58: 11-32) beschrieben. Allerdings handelt es sich bei diesem System um ein Flüssig-Membran-System mit den zuvor erwähnten Nachteilen, insbesondere hinsichtlich der geringen Stabilität der flüssigen Membranvesikel. Ein weiterer Nachteil ist, daß die Komponenten des verwendeten Extraktionsmittels für biologische Produktionssysteme, z.B. Fermentationen, toxisch sind, wodurch sich der Einsatzbereich dieses Verfahrens auf eine Batch-Extraktion beschränkt. Auch hier besteht somit nicht die Möglichkeit, einer potentiellen Produktinhibition durch eine integrierte Abtrennung und Aufarbeitung des Produktes vorzubeugen.

Wieczorek S. et al, (1998, Bioprocess Engineering, 18: 75-77) beschreibt die Verwendung eines Gemisches aus Tridodecylamin, Kerosin und Octanol als Extraktionsmittel. Das Ziel dieses Verfahrens ist nicht die Gewinnung von Aminosäuren, wie in den bisher aufgeführten Systemen, sondern die Abtrennung von Zitronensäure, einer Substanz, die keinen Stickstoff enthält, aus der Kulturbrühe des Pilzes Aspergillus niger mit Hilfe eines Anionen-selektiven Carriers. Nachteilig bei diesem System ist auch hier die hohe Toxizität des Extraktionsmittels. Eine Rückführung des Mediums in den Produktionsprozeß erfordert aufgrund dessen die Einführung zusätzlicher und aufwendiger Verfahrens- bzw. Reinigungsschritte, wodurch zudem nur eine teilweise Rückführung des Mediums realisiert werden kann.

In WO 93 23150 A wird ein Verfahren beschrieben, das als Donnan-Dialyse zu betrachten ist und wobei unter anderem organische Substanzen über eine, beidseitig mit wässriger Phase umgebene, selektive Membran getrennt werden mit Hilfe eines wasserlöslichen Extraktionsreagenzes.

Aufgabe der vorliegenden Erfindung ist es daher, ein Verfahren zur Abtrennung von einer oder mehreren in einem wäßrigen Gemisch vorhandenen organischen Substanzen enthaltend zumindest eine positiv geladene und/oder aufladbare stickstoffhaltige Gruppe mittels Extraktion über wenigstens eine poröse Membran zur Verfügung zu stellen, das die oben genannten Nachteile nicht mehr aufweist.

Gelöst wird diese Aufgabe dadurch, daß ein Extraktionsmittel eingesetzt wird, das Alkane, Alkene oder Fettsäureester, die 6 bis 20 C-Atome aufweisen, und wenigstens einen flüssigen Kationentauscher enthält, und eine Membran, die entweder von dem wässrigen Gemisch oder dem Extraktionsmittel benetzbar ist.

Der flüssige Kationentauscher hat dabei die Funktion eines Carriers. Die organischen Substanzen werden dabei erfindungsgemäß bevorzugt aus dem Extraktionsmittel in eine wässrige Phase rückextrahiert.

Die Alkane, Alkene oder Fettsäureester, die 6 bis 20 C-Atome aufweisen sind alle längerkettige organischen Verbindungen. Es werden vorzugsweise solche Verbindungen eingesetzt, die nur schwer mit Wasser mischbar oder nur schwer in Wasser lösbar sind und bei Temperaturen zwischen 10 und 60 °C, vorzugsweise zwischen 20 und 40 °C flüssig sind.

Erfindungsgemäß besonders bevorzugt sind solche Verbindungen mit 12 bis 18 C-Atomen. Denkbar sind hier verzweigte, unverzweigte, gesättigte, ungesättigte oder teilweise aromatische organische Verbindungen. Beispiele für erfindungsgemäß verwendbare längerkettige organische Verbindungen sind nicht nur solche Alkane, Alkene oder Fettsäureester, aber auch Gemische aus mehreren dieser Verbindungen. Diese Verbindungen haben bei den erfindungsgemäßen Verfahren die Funktion eines Lösungsmittels.

Als Alkane sind beispielsweise Hexan, Cyclohexan, Decan, Ethyl-Decan, Dodecan oder Gemische davon einsetzbar. Besonders bevorzugt ist Kerosin. Als Alkene sind z.B. Hexen, Nonen, Decen, Dodecen oder Gemische davon einsetzbar. Als Fettsäureester sind insbesondere Alkylstearate mit Alkylgruppen mit mehr als 2 C-Atomen einsetzbar. Beispiele für Fettsäureester sind Ethylstearat, Butylstearat, Isopropylstearat, Ethyl-Palmitat oder Butyl-Linoleat. Besonders bevorzugt ist der Einsatz von Kerosin oder Butylstearat. Zwei oder mehr der genannten organischen Verbindungen können auch in Form von Gemischen zum Einsatz kommen.

Als flüssige Kationentauscher werden vorzugsweise Ester aus anorganischen Säuren und organischen Resten, die vorzugsweise verzweigt sind, eingesetzt.
Zu den anorganischen Säuren zählen vorzugsweise Phosphorsäure, phosphorige Säure, Schwefelsäure und schweflige Säure. Ganz besonders bevorzugt ist Phosphorsäure. Die erfindungsgemäß verwendeten organischen Reste sind vorzugsweise verzweigte und/oder unverzweigte Alkyl- oder Alkenyl-Gruppen mit wenigstens 4 C-Atomen, vorzugsweise 4 bis 20 C-Atomen. Zu den bevorzugten flüssigen Kationentauschem zählen Di-2-Ethyl-Hexyl-Phosphorsäure-Ester, Mono-2-Ethyl-Hexyl-Phosphorsäure-Ester, Di-Nonyl-Naphthalen-Sulfonsäure-Ester oder Gemische hiervon.
Erfindungsgemäß bevorzugt ist das Gemisch aus Di-2-Ethyl-Hexyl-Phosphorsäure-Ester und Mono-2-Ethyl-Hexyl-Phosphorsäure-Ester. Der Gehalt an Mono-2-Ethyl-Hexyl-Phosphorsäure-Ester in diesem Gemisch liegt vorzugsweise über 40 Gew.%, besonders bevorzugt über 80 Gew.% bezogen auf den Gesamtanteil an flüssigem Kationentauscher.
Die beschriebenen flüssigen Kationentauscher sind vorzugsweise mit einem Anteil von 2 bis 25 Gew.% bezogen auf die Menge an Alkane, Alkene oder Fettsäureester mit 6 bis 20 C-Atomen in dem Extraktionsmittel enthalten. Ganz besonders bevorzugt sind Anteile von 5 bis 20 Gew.% an flüssigem Kationentauscher und höchst bevorzugt sind Anteile von 8 bis 15 Gew.% an flüssigem Kationentauscher.
Das Extraktionsmittel kann erfindungsgemäß neben den genannten Verbindungen auch weitere Substanzen enthalten. Hierzu zählen auch nach dem Stand der Technik bekannte Extraktionsmittel.

Für das erfindungsgemäße Verfahren wird bevorzugt eine poröse Membran, die entweder von dem wässrigen Gemisch oder dem Extraktionsmittel benetzbar ist, eingesetzt, die eine Porendichte von 5 bis 95% aufweist. Besonders bevorzugt sind Porendichten von ≥ 30%. Höchst bevorzugt sind Porendichten von ≥ 40%. Grundsätzlich können für das erfindungsgemäße Verfahren die nach dem Stand der Technik bekannten porösen Membranen, die entweder von dem wässrigen Gemisch oder dem Extraktionsmittel benetzbar sind, eingesetzt werden. Bevorzugt ist hierbei die Verwendung von Membranen mit einer möglichst hohen Porendichte. Zu den ganz besonders bevorzugten Membranen zählen die Hohlfaserkontaktoren.

Vorzugsweise werden in den erfindungsgemäßen Verfahren poröse Membranen eingesetzt, die eine Porengröße von ≤ 2 µm aufweisen. Besonders bevorzugt sind Membranen mit einer Porengröße von ≤ 1 µm. Ganz besonders sind Porengrößen von ≤ 0, 5 µm. Höchst bevorzugt sind Porengrößen von ≤ 0, 05 µm.

Das erfindungsgemäße Verfahren eignet sich insbesondere für die Extraktion organischer Substanzen, die zumindest eine positiv geladene und/oder aufladbare stickstoffhaltige Gruppe enthalten. Ganz besonders bevorzugt eignet sich das erfindungsgemäße Verfahren für die Extraktion organischer Substanzen ausgewählt aus der Gruppe aliphatischer und/oder aromatischer Aminosäuren und/oder Lactame, deren Salze, Derivate oder Di- oder Oligopeptide oder Gemische dieser Verbindungen.

Zu den extrahierbaren Substanzen zählen beispielsweise L-Aminosäuren oder D-Aminosäuren. Grundsätzlich sind sowohl natürliche als auch nicht natürliche Aminosäuren extrahierbar. Denkbar sind alle D- und L-Formen essentieller Aminosäuren. Beispiele für extrahierbare Aminosäuren sind L-Phenylalanin, D-Phenylalanin, L-Tryptophan, D-Tryptophan, L-Tyrosin, D-Tyrosin, D-p-Hydroxy-Phenylglycin, D-Phenylglycin, Di-Hydroxy-Phenylalanin.
Weiterhin sind mit dem erfindungsgemäßen Verfahren Lactame extrahierbar. Hierzu zählen u. a. β-Lactame, Caprolactam, Penicillin G.
Ferner können erfindungsgemäß auch Peptide, insbesondere aber Di- oder Oligopeptide extrahiert werden. Hierzu zählt beispielsweise L-Aspartyl-L-Phenylalanin als Vorläufermolekül für die Herstellung von Aspartam.
Extrahierbar sind auch Aminoalkohole, z.B. 1S, 2R-cis-(-)-Aminoindanol.
Die erfindungsgemäße Extraktion ist außerdem anwendbar für die Gewinnung von Aminen oder Amiden.

Ein bevorzugtes Einsatzgebiet des erfindungsgemäßen Verfahrens ist die Extraktion aus Fermentationslösungen, Abwässern und/oder wäßrigen Gemischen chemischer Synthese- und/oder Degradationsprozesse. Insbesondere läßt sich das erfindungsgemäße Verfahren in Fermentationsprozesse integrieren. Die Fermentationsprozesse können aerob oder anaerob ablaufen und als batch-, semikontinuierliche oder kontinuierliche Prozesse betrieben werden.

Die Erfindung betrifft ferner ein Verfahren, bei dem die folgenden Schritte durchgeführt werden:
a) das wäßrige Gemisch wird aus einem Behälter entnommen,
b) über eine erste poröse Membran geführt wird, die entweder von dem wässrigen Gemisch oder dem Extraktionsmittel, das Alkane, Alkene oder Fettsäureester, die 6 bis 20 C-Atome aufweisen, und wenigstens einen flüssigen Kationentauscher enthält, benetzbar ist,
c) mittels des Extraktionsmittels extrahiert,
d) das wässrige Retentat wird in den Behälter zurückgeführt,
e) die extrahierten organischen Substanzen werden über eine zweite poröse Membran geführt, die entweder von dem wässrigen Gemisch oder dem Extraktionsmittel benetzbar ist und
f) dort in eine wässrige Phase rückextrahiert.

Vorzugsweise ist das Verfahren so ausgestaltet, daß in Stufe d) eine vollständige Rückführung des wässrigen Retentats in den Behälter erfolgt. Ebenfalls bevorzugt ist, daß die Druckdifferenz zwischen dem wässrigen Gemisch und dem Extraktionsmittel 0,1 bis 10 bar beträgt. Besonders bevorzugt sind Druckdifferenzen von 0,5 bis 5 bar, ganz besonders bevorzugt sind Druckdifferenzen von 2 bis 3 bar.

Eine besonders bevorzugte Ausführungsform des erfindungsgemäßen Verfahrens ist, daß die Rückextraktion unter gleichzeitiger Aufkonzentrierung der organischen Substanzen erfolgen kann. Ganz besonders bevorzugt ist die Rückextraktion unter gleichzeitiger Aufkonzentrierung der organischen Substanzen.

Ein besonders bevorzugtes Anwendungsgebiet der vorliegenden Erfindung ist die Extraktion von Substanzen aus Fermentationslösungen. Die Fermentationen können aerob oder anaerob und als batch-, serni-kontinuierliche oder kontinuierliche Prozesse betrieben werden. Vorzugsweise ist das Verfahren so gestaltet, daß die Extraktion kontinuierlich und simultan zu einer in Behälter 1 ablaufenden Reaktion, wie beispielsweise einer Fermentation, d.h. also integriert, erfolgt. Als besondere Ausführungsform des erfindungsgemäßen Verfahrens kann auch die Rückextraktion der organischen Substanzen in die wässrige Phase als integrierter Prozeß zu einer in Behälter 1 ablaufenden Reaktion erfolgen. Es wird jedoch darauf hingewiesen, daß der Gegenstand der Erfindung auf derartige Prozesse nicht beschränkt ist.

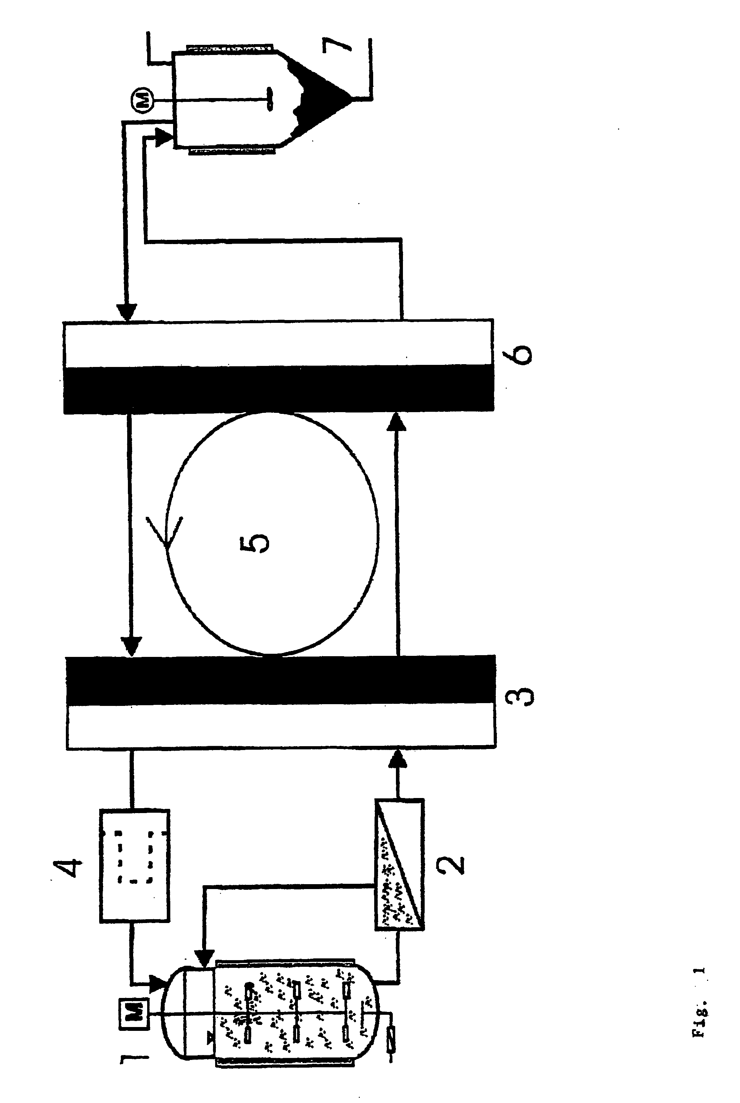

Im folgenden wird anhand der Figur 1 die Anwendung des erfindungsgemäßen Verfahrens anhand eines integrierten Fermentationsprozesses näher erläutert:

Aus dem Fermenter 1 wird kontinuierlich Fermentationslösung entnommen und über ein Ultrafiltrations-Hohlfasermodul 2 abgeführt. In dem Hohlfaserkontaktor 3 wird die organische Substanz, die aus der Fermentationslösung abgetrennt werden soll, in das Extraktionsmittel 5 extrahiert. Das Extraktionsmittel 5 enthält erfindungsgemäß zumindest teilweise längerkettige organischen Verbindungen und wenigstens einen flüssigen Kationentauscher.
Das wässrige Retentat in dem Hohlfaserkontaktor 3 wird über einen Sterilfilter 4 in den Fermenter 1 zurückgeführt.
Die extrahierte organische Substanz wird über den Hohlfaserkontaktor 6 geführt und gegebenenfalls unter gleichzeitiger Aufkonzentrierung in eine wässrige Phase 7 rückextrahiert. Aus dieser wässrigen Phase 7 kann durch Aufgabenstellung die Substanz beispielsweise auskristallisiert werden. Das Ultrafiltrations-Hohlfasermodul 2 und der Sterilfilter 4 können im Bedarsfall auch weggelassen werden.

Ein wesentlicher Vorteil des erfindungsgemäßen Verfahrens ist die selektive Gewinnung der gewünschten organischen Substanzen bei gegebenenfalls gleichzeitiger Aufkonzentrierung. Die Aufkonzentrierung der organischen Substanzen erfolgt erfindungsgemäß bei der Rückextraktion in die wässrige Phase. Der besondere Vorteil des beschriebenen Verfahrens liegt darin, daß Fermentation und Extraktion in einem integrierten Verfahren durchgeführt werden können. D.h., während eines batch-, semi-kontinuierlich oder kontinuierlich laufenden Fermentationsprozesses kann durch eine kontinuierlich und simultan erfolgende Extraktion eine Gewinnung der gewünschten organischen Substanzen erreicht werden. Zugleich ist es möglich, die nicht extrahierten Teile der Fermentationslösung (wässriges Retentat) in den Fermentationsprozeß zurückzuführen. Eine Endprodukthemmung der zellspezifischen Produktion wird somit ausgeschlossen. Das gewählte Extraktionssystem ist dabei nicht toxisch und aufgrund eines frei wählbaren pH-Wertes sehr umweltschonend. Prinzipiell besteht außerdem die Möglichkeit, die noch biomassehaltige Fermentationslösung direkt und ohne vorherige Zellseparation in das Extraktionsmodul einzuleiten, wodurch ein zusätzlicher Prozeßschritt eingespart wird.

Beispielhaft ist im folgenden die selektive Abtrennung von L-Phenylalanin aus einer Modell-Lösung näher beschrieben:
Die eingesetzte Modell-Lösung repräsentiert eine typische Zusammensetzung eines Kulturmediums einer mikrobiellen Fermentation. Folgende Konzentrationen an Kationen (Firma Merck) und Aminosäuren (Firma Fluka) wurden durch die Einwaage geeigneter Salze in Wasser eingestellt:

Für die Extraktion des L-Phenylalanins wurden 10 Liter Modell-Lösung zuvor genannter Zusammensetzung mit einem Volumenstrom von 250 l/h bei pH 7 und 30 °C Vorlauftemperatur durch den ersten Hohlfaserkontaktor (Hohlfasermodul Typ Celgard Polypropylen, X30 240 ID der Firma Hoechst Celanese AG) gepumpt. Der Druck beim Eingang in den Kontaktor lag bei 2,2 bar und bei 0,8 bar bei Austritt aus dem Kontaktor.
Das Extraktionsmittel bestand zu 10 Vol.-% aus Di-2-Ethyl-Hexyl-Phosphorsäure-Ester (Firma Fluka) gelöst in Kerosin (Firma Sigma Aldrich). Das eingesetzte Volumen des Extraktionsmittels umfaßte 7,5 Liter. Im Gegenstrom zu der wässrigen Phase der Modell-Lösung wurde die organische Phase des Extraktionsmittels mit einem Volumenstrom von 156 l/h in einem Kreislauf durch zwei nacheinander geschaltete Hohlfaserkontaktoren gepumpt. Der Druck am Eingang des ersten Hohlfaserkontaktors lag bei 0,3 bar und am Kontaktorausgang bei 0,2 bar. Am Eingang des zweiten Hohlfaserkontaktors herrschte ebenfalls ein Druck von 0,2 bar und am Ausgang des zweiten Hohlfaserkontaktors lagen 0,1 bar Druck vor. Das im Zuge der Extraktion in die organische Phase des Extraktionsmittels überführte L-Phenylalanin wurde anschließend in eine wässrige Phase rückextrahiert. Dabei wurden 6 l dieser wässrigen Phase eingesetzt, die aus einer H₂SO₄-Lösung (Firma Fluka) mit einer Konzentration von 1 mol/l und einem pH-Wert von 0 bestand. Diese wässrige Phase wurde mit einem Volumenstrom von 500 l/h im Gegenstrom zu der organischen Extraktionsmittelphase durch den zweiten Hohlfaserkontaktor gepumpt. Dabei herrschte am Eingang des Hohlfaserkontaktors ein Druck von 1,2 bar und am Ausgang von 0,5 bar.
Die Selektivität der Extraktion in Bezug auf die Kationen und Aminosäuren betrug 95% oder mehr. Dabei wurde eine Aufkonzentrierung des L-Phenylalanins in der wässrigen Phase um 20% bis zu einem Bereich von 400 bis 500% im Vergleich zur eingesetzten Menge in der Modell-Lösung ermöglicht
Ein weiterer positiver Effekt des erfindungsgemäßen Verfahrens besteht darin, daß durch den Eintrag von flüssigem Kationentauscher in die Modell-Lösung während der Reaktion in dem Behälter 1 die Menge an gebildeter organischer Substanz um mindestens 40% gegenüber der Modell-Lösung ohne flüssigen Kationentauscher gesteigert werden kann.

## Patentansprüche

1. Verfahren zur Abtrennung von einer oder mehreren in einem wäßrigen Gemisch vorhandenen organischen Substanzen enthaltend zumindest eine positiv geladene und/oder aufladbare stickstoffhaltige Gruppe mittels Extraktion über wenigstens eine poröse Membran **dadurch gekennzeichnet, daß** ein Extraktionsmittel, das Alkane, Alkene oder Fettsäureester, die 6 bis 20 C-Atome aufweisen, und wenigstens einen flüssigen Kationentauscher enthält, und eine Membran, die entweder von dem wässrigen Gemisch oder dem Extraktionsmittel benetzbar ist, eingesetzt wird.

2. Verfahren nach Anspruch 1 **dadurch gekennzeichnet, daß** die organischen Substanzen aus dem Extraktionsmittel in eine wässrige Phase rückextrahiert werden.

3. Verfahren nach einem der Ansprüche 1 oder 2 **dadurch gekennzeichnet, daß** Alkane, Alkene oder Fettsäureester mit 12 bis 18 C-Atomen eingesetzt werden.

4. Verfahren nach einem der Ansprüche 1 bis 3 **dadurch gekennzeichnet, daß** es als flüssigen Kationentauscher Ester aus anorganischen Säuren und organischen Resten, die vorzugsweise verzweigt sind, enthält.

5. Verfahren nach einem der Ansprüche 1 bis 4 **dadurch gekennzeichnet, daß** als flüssiger Kationentauscher Ester aus Phosphorsäure, phosphoriger Säure, Schwefelsäure oder schwefliger Säure eingesetzt werden.

6. Verfahren nach einem der Ansprüche 1 bis 5 **dadurch gekennzeichnet, daß** es als flüssigen Kationentauscher Di-2-Ethyl-Hexyl-Phosphorsäure-Ester, Mono-2-Ethyl-Hexyl-Phosphorsäure-Ester, Di-Nonyl-Naphthalen-Sulfonsäure-Ester oder Gemische hiervon enthält.

7. Verfahren nach einem der Ansprüche 1 bis 6 **dadurch gekennzeichnet, daß** es bezogen auf die Menge an Alkane, Alkene oder Fettsäureester mit 6 bis 20 C-Atomen 2 bis 25 Gew.%, vorzugsweise 5 bis 20 Gew.%, besonders bevorzugt 8 bis 15 Gew.% flüssigen Kationentauscher enthält.

8. Verfahren nach einem der Ansprüche 1 bis 7 **dadurch gekennzeichnet, daß** organische Substanzen ausgewählt aus der Gruppe aliphatischer und/oder aromatischer Aminosäuren und/oder Lactame, deren Salze, Derivate oder Di- oder Oligopeptide oder Gemische dieser Verbindungen extrahiert werden.

9. Verfahren nach einem der Ansprüche 1 bis 8 **dadurch gekennzeichnet, daß** eine poröse Membran eingesetzt wird, die eine Porendichte von 5 bis 95%, vorzugsweise ≥ 30% aufweist.

10. Verfahren nach einem der Ansprüche 1 bis 9 **dadurch gekennzeichnet, daß** eine poröse Membran eingesetzt wird, die eine Porendichte von ≥ 40% aufweist.

11. Verfahren nach einem der Ansprüche 1 bis 10 **dadurch gekennzeichnet, daß** als poröse Membran ein Hohlfaserkontaktor eingesetzt wird.

12. Verfahren nach einem der Ansprüche 1 bis 11 **dadurch gekennzeichnet, daß**
a) das wäßrige Gemisch aus einem Behälter (1) entnommen wird,
b) über eine erste poröse Membran (3), die entweder von dem wässrigen Gemisch oder dem Extraktionsmittel (5), das Alkane, Alkene oder Fettsäureester, die 6 bis 20 C-Atome aufweisen, enthält, benetzbar ist, geführt wird,
c) mittels des Extraktionsmittels (5) extrahiert wird,
d) das wässrige Retentat in den Behälter (1) zurückgeführt wird,
e) die extrahierten organischen Substanzen über eine zweite poröse Membran (6), die entweder von dem wässrigen Gemisch oder dem Extraktionsmittel benetzbar ist, geführt werden und
f) dort in eine wässrige Phase (7) rückextrahiert werden.

13. Verfahren nach Anspruch 12 **dadurch gekennzeichnet, daß** in Stufe d) eine vollständige Rückführung des wässrigen Retentats in den Behälter (1) durchgeführt wird.

14. Verfahren nach einem der Ansprüche 12 oder 13 **dadurch gekennzeichnet, daß** die Druckdifferenz zwischen dem wässrigen Gemisch und dem Extraktionsmittel 0,1 bis 10 bar, vorzugsweise 0,5 bis 5 bar, besonders bevorzugt 2 bis 3 bar beträgt.

15. Verfahren nach einem der Ansprüche 12 bis 14, **dadurch gekennzeichnet, daß** die Rückextraktion unter gleichzeitiger Aufkonzentrierung der organischen Substanzen erfolgt.

16. Verfahren nach einem der Ansprüche 12 bis 15 **dadurch gekennzeichnet, daß** die Extraktion kontinuierlich und simultan zu einer in Behälter (1) ablaufenden Reaktion erfolgt.

17. Verfahren nach einem der Ansprüche 1 bis 16 **dadurch gekennzeichnet, daß** aus Fermentationslösungen, Abwässern und/oder wäßrigen Gemischen chemischer Synthese- und/oder Degradationsprozesse extrahiert wird.

## Claims

1. Process for separating from an aqueous mixture one or more organic substances containing at least one positively charged and/or chargeable nitrogenous group by means of extraction via at least one porous membrane, **characterized in that** use is made of an extraction agent which contains and at least one liquid cation exchanger, and of a membrane that is wettable by either the aqueous mixture or by the extraction agent.

2. Process according to Claim 1, **characterized in that** the organic substances are re-extracted from the extraction agent into an aqueous phase.

3. Process according to either Claim 1 or 2, **characterized in that** relatively long-chain organic compounds with 6 to 20 C atoms, preferably 12 to 18 C atoms, are employed.

4. Process according to any one of Claims 1 to 3, **characterized in that** esters of inorganic acids and organic, preferably branched, residue groups are employed as liquid cation exchangers.

5. Process according to any one of Claims 1 to 4, **characterized in that** esters of phosphoric acid, phosphorous acid, sulphuric acid or sulphurous acid are employed as liquid cation exchangers.

6. Process according to any one of Claims 1 to 5, **characterized in that** di-2-ethylhexyl phosphoric acid esters, mono-2-ethylhexyl phosphoric acid esters, dinonylnaphthalene sulphonic acid esters or mixtures thereof are employed as liquid cation exchangers.

7. Process according to any one of Claims 1 to 6, **characterized in that** liquid cation exchangers are employed in an amount of 2 to 25 wt.%, preferably 5 to 20 wt.%, most preferably 8 to 15 wt.%, relative to the amount of alkanes, alkenes or fatty acid with 6 to 20 C-atoms.

8. Process according to any one of Claims 1 to 7, **characterized in that** organic substances belonging to the group of aliphatic and/or aromatic amino acids and/or lactams, the salts, derivatives or di- or oligopeptides thereof or mixtures of these compounds are extracted.

9. Process according to any one of Claims 1 to 8, **characterized in that** a porous membrane having a pore density of 5 to 95%, preferably ≥ 30%, is employed.

10. Process according to any one of Claims 1 to 9, **characterized in that** a porous membrane having a pore density of ≥ 40%, is employed.

11. Process according to any one of Claims 1 to 10, **characterized in that** a hollow-fibre contactor is employed as porous membrane.

12. Process according to any one of claims 1 to 11, **characterized in that**
a) the aqueous mixture is drawn from a reservoir (1),
b) led across a first porous membrane (3) which is wettable by either the aqueous mixture or by an extraction agent (5) which contains alkanes, alkenes or fatty acid esters with 6 to 20 C-atoms and at least one liquid cation exchanger,
c) extracted with the extraction agent (5),
d) the aqueous retentate (1) is returned to the reservoir,
e) the extracted organic substances are led across a second porous membrane (6) which is wettable by either the aqueous mixture or by the extraction agent and
f) there re-extracted into an aqueous phase (7).

13. Process according to claim 12, **characterized in that** in stage d) the aqueous retentate is completely returned to reservoir (1).

14. Process according to either Claim 12 or 13, **characterized in that** the pressure difference between the aqueous mixture and the extraction agent lies between 0.1 and 10 bar, preferably between 0.5 and 5 bar, most preferably between 2 and 3 bar.

15. Process according to any one of Claims 12 to 14, **characterized in that** the re-extraction is effected with simultaneous concentration augmentation of the organic substances.

16. Process according to any one of Claims 12 to 15, **characterized in that** the extraction takes place continuously and simultaneously with a reaction that is proceeding in reservoir 1.

17. Process according to any one of Claims 1 to 16, **characterized in that** extraction from fermentation solutions, effluent flows and/or aqueous mixtures from chemical synthesis and/or degradation processes is effected.

## Revendications

1. Procédé de séparation d'une ou plusieurs substances organiques présentes dans un mélange aqueux et contenant au moins un groupement azoté positivement chargé et/ou susceptible d'être chargé, au moyen d'une extraction à travers au moins une membrane poreuse, **caractérisé en ce qu'**un agent d'extraction, qui contient des alcanes, des alcènes ou des esters d'acide gras qui comprennent 6 à 20 atomes de carbone et au moins un échangeur de cations liquide, est employé, ainsi qu'une membrane qui est mouillable soit par le mélange aqueux, soit par l'agent d'extraction.

2. Procédé selon la revendication 1, **caractérisé en ce que** les substances organiques sont réextraites de l'agent d'extraction dans une phase aqueuse.

3. Procédé selon l'une quelconque des revendications 1 ou 2, **caractérisé en ce que** des alcanes, des alcènes ou des esters d'acides avec 12 à 18 atomes de carbone sont employés.

4. Procédé selon l'une quelconque des revendications 1 à 3, **caractérisé en ce que** des esters d'acides inorganiques et de résidus organiques de préférence ramifiés sont employés comme échangeurs de cations liquides.

5. Procédé selon l'une quelconque des revendications 1 à 4, **caractérisé en ce que** des esters d'acides phosphoriques, d'acides phosphoreux, d'acides sulfuriques et d'acides sulfureux sont employés comme échangeurs de cations liquides.

6. Procédé selon l'une quelconque des revendications 1 à 5, **caractérisé en ce que** des esters de l'acide di-2-éthyl-hexyl-phosphorique, des esters d'acide mono-2-éthyl-hexyl-phosphorique, des esters d'acide di-nonyl-naphtalène-sulfonique ou des mélanges de ces composés sont employés comme échangeurs de cations liquides.

7. Procédé selon l'une quelconque des revendications 1 à 6, **caractérisé en ce que** par rapport à la quantité d'alcanes, d'alcènes ou d'esters d'acides gras avec 6 à 20 atomes de carbone, de 2 à 25 % en poids, de préférence entre 5 et 20 % en poids, et encore plus préférentiellement entre 8 et 15 % en poids d'échangeurs de cations liquides sont contenus.

8. Procédé selon l'une quelconque des revendications 1 à 7, **caractérisé en ce que** des substances organiques, choisies dans le groupe comprenant des acides aminés et/ou des lactames aliphatiques et/ou aromatiques, les sels, dérivés ou di- ou oligopeptides de ces composés ou des mélanges de ces composés, sont extraites.

9. Procédé selon l'une quelconque des revendications 1 à 8, **caractérisé en ce qu'**une membrane poreuse est employée, laquelle comporte une densité de porosité comprise entre 5 et 95 %, de préférence ≥ 30 %.

10. Procédé selon l'une quelconque des revendications 1 à 9, **caractérisé en ce qu'**une membrane poreuse est employée, laquelle comporte une densité de porosité ≥ 40 %.

11. Procédé selon l'une quelconque des revendications 1 à 10, **caractérisé en ce qu'**un contacteur à fibres creuses est employé comme membrane poreuse.

12. Procédé selon l'une quelconque des revendications 1 à 11, **caractérisé en ce que**
a) le mélange aqueux est prélevé d'un réservoir (1),
b) est conduit à travers une première membrane poreuse (3) qui est mouillable soit par le mélange aqueux, soit par l'agent d'extraction (5) qui contient des alcanes, des alcènes ou des esters d'acides gras qui comprennent 6 à 20 atomes de carbone,
c) est extrait au moyen d'un agent d'extraction (5),
d) le rétentat aqueux est ramené vers le réservoir (1),
e) les substances organiques extraites sont conduites à travers une deuxième membrane poreuse (6) qui est mouillable soit par le mélange aqueux soit par l'agent d'extraction (5), et
f) sont alors réextraites dans une phase aqueuse (7).

13. Procédé selon la revendication 12, **caractérisé en ce que** dans l'étape d) le rétentat aqueux est totalement ramené vers le réservoir (1).

14. Procédé selon l'une quelconque des revendications 12 ou 13, **caractérisé en ce que** la différence de pression entre le mélange aqueux et l'agent d'extraction est comprise entre 0,1 et 10 bar, de préférence entre 0,5 et 5 bar, et encore plus préférentiellement entre 2 et 3 bar.

15. Procédé selon l'une quelconque des revendications 12 à 14, **caractérisé en ce que** la réextraction est simultanée à une augmentation de la concentration des substances organiques.

16. Procédé selon l'une quelconque des revendications 12 à 15, **caractérisé en ce que** l'extraction a lieu de façon continue et simultanément à une réaction se déroulant dans le réservoir (1).

17. Procédé selon l'une quelconque des revendications 1 à 16, **caractérisé en ce que** l'extraction est effectuée à partir de solutions de fermentation, d'eaux usées et/ou de mélanges aqueux issus de procédés chimiques de synthèse et/ou de dégradation.
